# EUROPEAN PATENT SPECIFICATION

(11) **EP 4 437 936 B1**
(45) Date of publication and mention of the grant of the patent: **04.11.2026**
(21) Application number: 24163742.0
(22) Date of filing: 15.03.2024
(51) Int. Cl.: A61B 3/00, A61B 3/10, A61B 3/14, A61B 3/15

(54) **OPHTHALMOLOGICAL APPARATUS**
OPHTHALMOLOGISCHES GERÄT
APPAREIL OPHTALMOLOGIQUE

(30) Priority: 28.03.2023 JP 2023050908
(43) Date of publication of application: 02.10.2024
(73) Proprietor: Topcon Corporation, Tokyo 174-8580 (JP)
(72) Inventor: KATO, Yuichi, Tokyo, 174-8580 (JP); OOHARA, Ryuichi, Tokyo, 174-8580 (JP)
(74) Representative: Louis Pöhlau Lohrentz

(56) References cited:
- EP-A1- 2 090 221
- US-A1- 2019 254 514
- US-A1- 2020 100 673
- US-A1- 2022 151 483

## Description

### FIELD OF THE INVENTION

The present disclosure relates to an ophthalmological apparatus.

### BACKGROUND

Conventionally, there has been disclosed an ophthalmologic system equipped with an ophthalmological apparatus body having a measuring device, a capturing device or a treating device of a subject eye, and a chin support, and peripheral equipment such as an optical table and a chair (see JP2005-013472A). In this system, based on the positional information of the examinee at the last measurement, the height of the chin support, the optical table, or the chair is adjusted, and a focus lens is moved such that the focus lens is in focus.

However, for example, if a certain period has elapsed since the last measurement, even the shift of each part to the last position may not have been necessarily appropriate due to eyesight change, disease progression, growth or aging of the examinee, etc. As a result, it was necessary to readjust the position of each part to be at a proper position, and it took a long time to obtain ocular information. US 2022/151483 A1 discloses an ophthalmic apparatus comprising an optical head unit including an optical system arranged to irradiate an eye to be examined with light and detect return light from the eye to be examined; an information obtaining unit configured to, using a learned model obtained by using information of a position relating to at least one of an eye to be examined and an optical head unit, obtain information of a position relating to at least one of an eye to be examined and the optical head unit from an image relating to an eye to be examined that is obtained using the optical head unit. US 2019/254514 A1 discloses a method for examining an eye of a patient by the patient themselves by means of an ophthalmological apparatus, said apparatus having front optics and an apparatus pupil. According to said method, the patient positions the ophthalmological apparatus relative to the eye, a measure of the deviation of the pupil of the eye from the apparatus pupil is determined, and a pupil correction signal is produced depending on the measure of the deviation, said pupil correction signal specifying a direction and/or a degree of the deviation and being output to the patient. EP 2 090 221 A1 discloses an ophthalmologic apparatus that has a setting part configured to acquire position information of a first examining part at the time of execution of a first examination and set a movable range of a second examining part in a direction of a second working distance based on the position information and distance information. Accordingly, it is possible to set an accurate movable range with respect to the position of the eye of a subject, and it is also possible to increase safety by preventing the examining part from coming in contact with the subject.

The present disclosure has been made by considering the above problem. An object of the present disclosure is to provide an ophthalmological apparatus that can efficiently acquire ocular information by shortening the period of time required for positional adjustment of each part of the ophthalmological apparatus.

### SUMMARY

To achieve the object, an ophthalmological apparatus according to claim 1 is provided.

### EFFECT OF THE INVENTION

The ophthalmological apparatus of the present disclosure, having the above configuration, can efficiently acquire ocular information by shortening the period of time required for positional adjustment of each part of the ophthalmological apparatus.

### BRIEF DESCRIPTION OF DRAWINGS

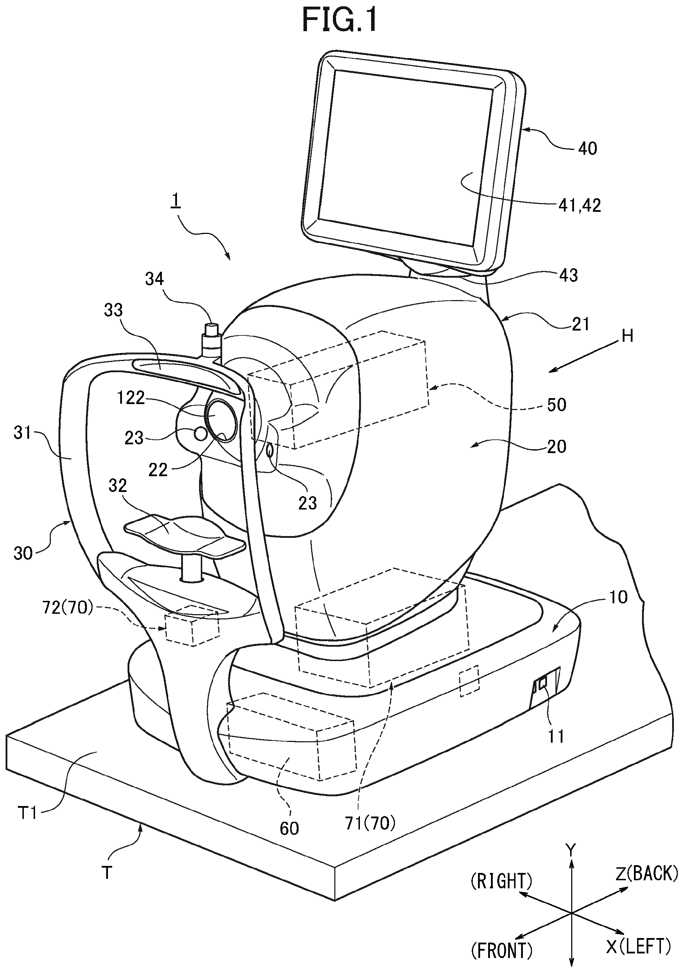

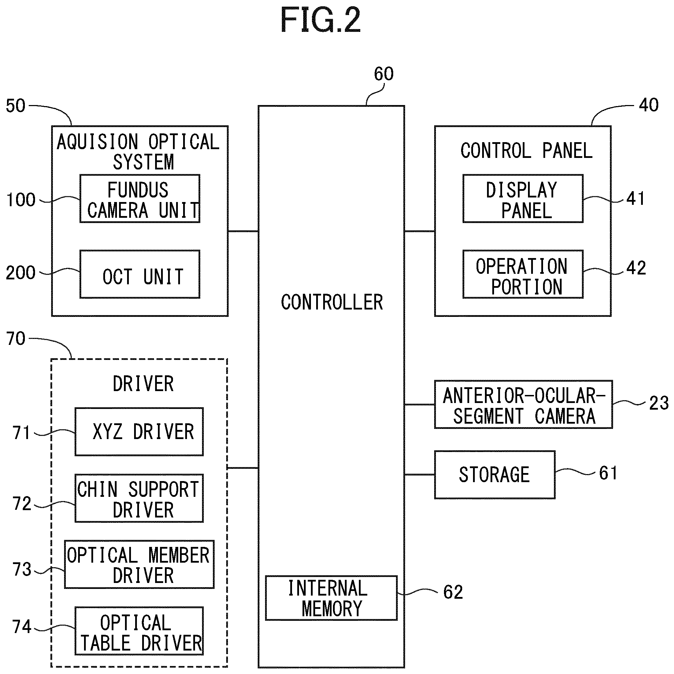

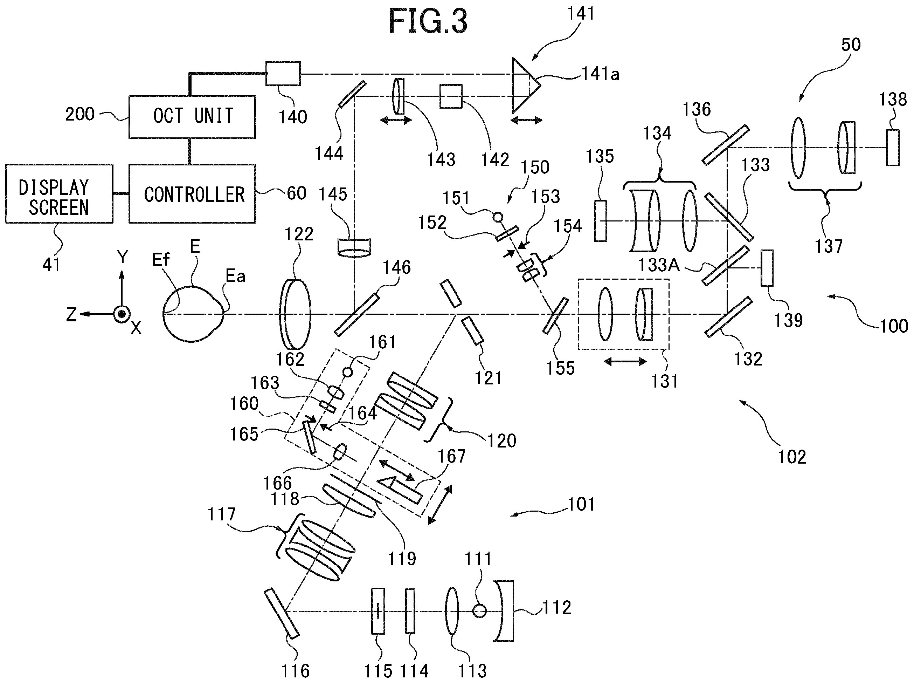

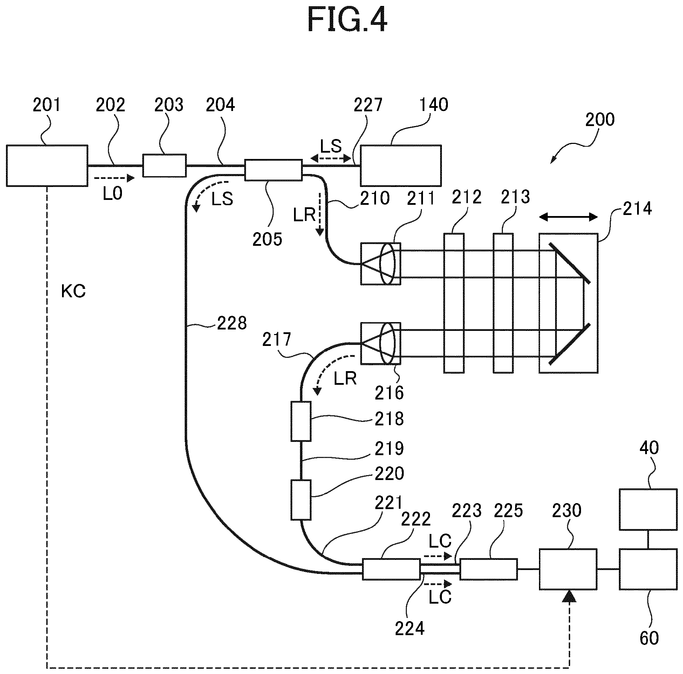

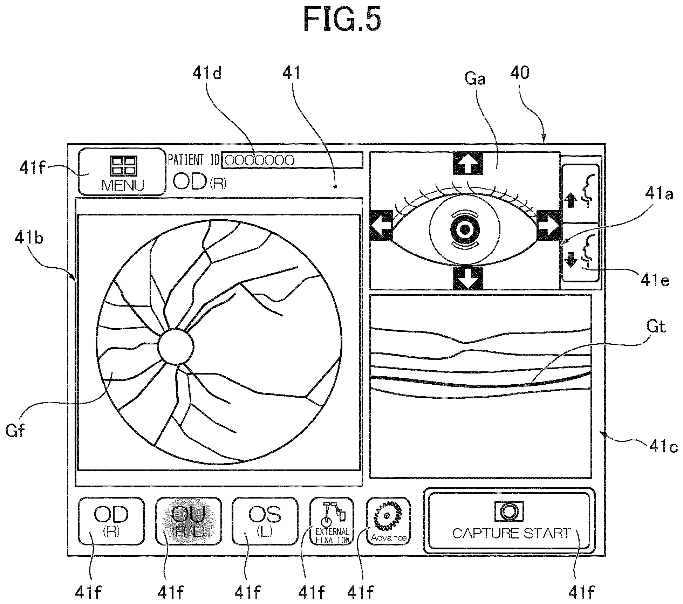

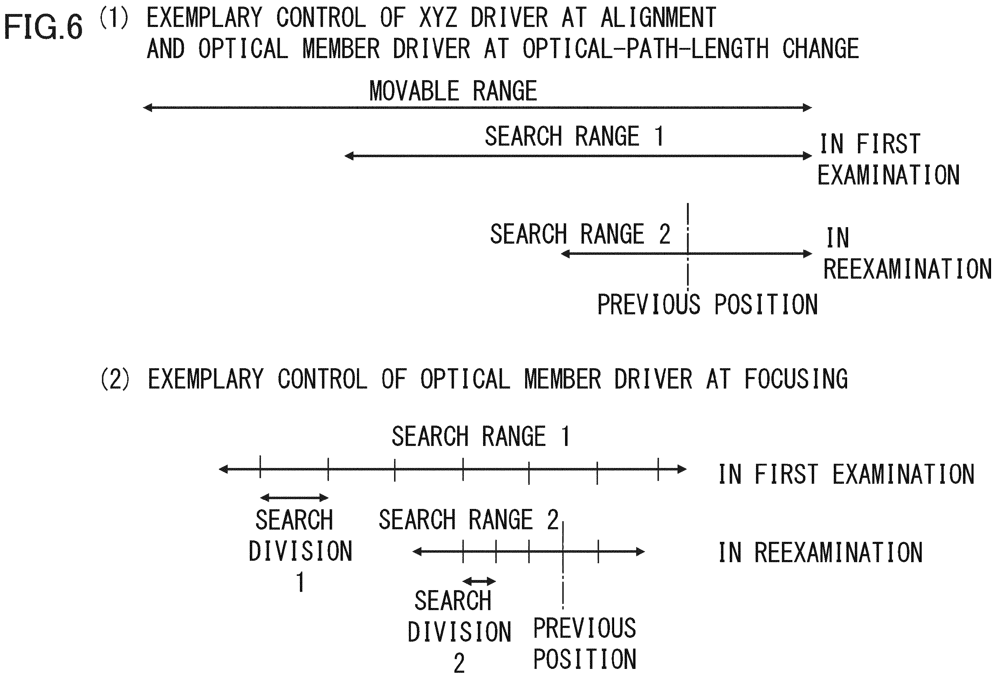

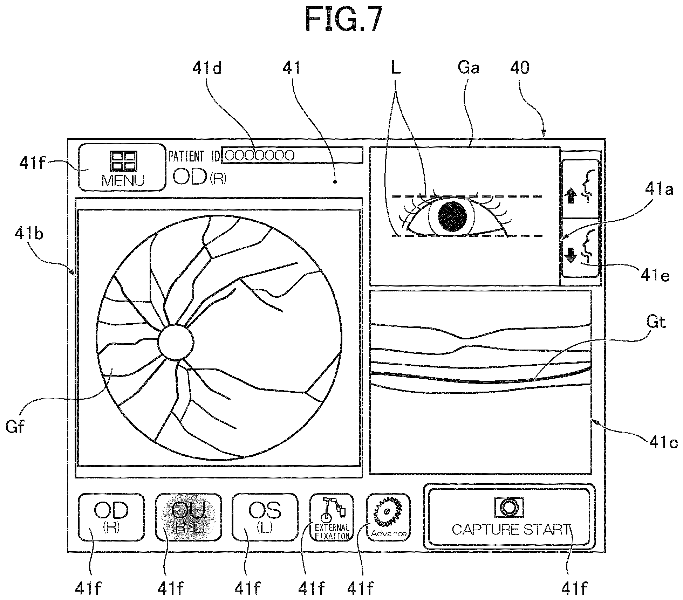

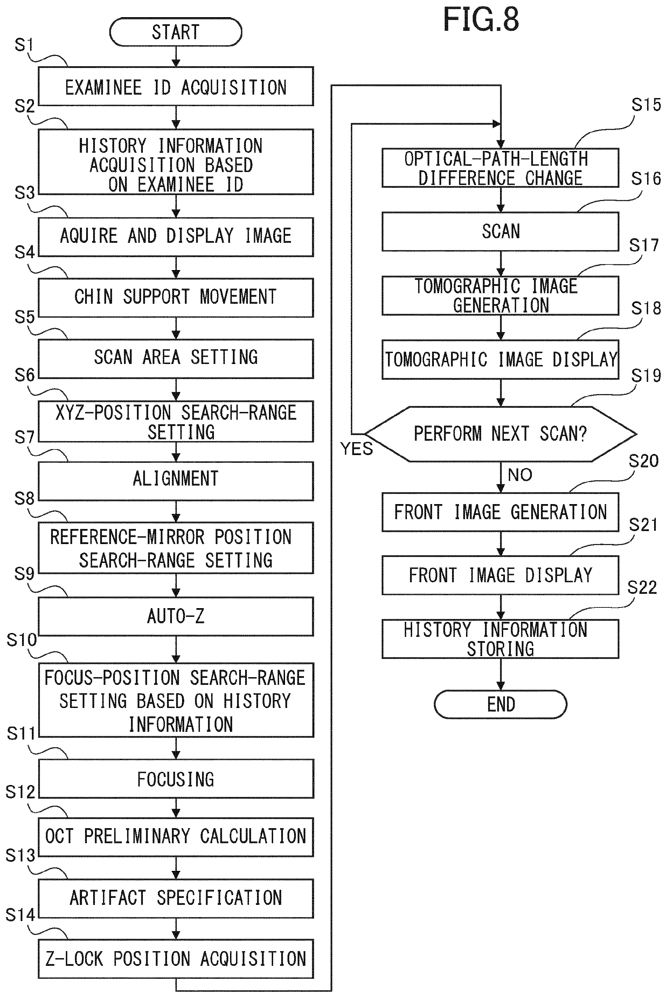

FIG. 1 is a perspective view illustrating the entire configuration of an ophthalmological apparatus according to a first embodiment. FIG. 2 is a block diagram illustrating a control configuration of the ophthalmological apparatus according to the first embodiment. FIG. 3 is a view illustrating a detailed configuration of an acquisition optical system of the ophthalmological apparatus according to the first embodiment. FIG. 4 is a view illustrating a detailed configuration of an OCT unit of the ophthalmological apparatus according to the first embodiment. FIG. 5 is a view illustrating one example of a display screen of the ophthalmological apparatus according to the first embodiment. FIG. 6 is a view illustrating a relationship between a movable range, a first search range at the first examination, and a second search range at a reexamination, without and with division into search divisions. FIG. 7 is a view illustrating another example of the display screen of the ophthalmological apparatus according to the first embodiment. FIG. 8 is a flowchart illustrating one example of the operation of the ophthalmological apparatus according the first embodiment.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for sake of clarity.

An ophthalmological apparatus according to a first embodiment of the present disclosure will be described below with reference to FIGS. 1 to 4. The ophthalmological apparatus according to the first embodiment is one that acquires ocular information of one subject eye at one time, but it is not limited thereto. The present disclosure can also be applied to an ophthalmological apparatus that is equipped with a pair of acquisition optical systems corresponding to the left and right subject eyes, to acquire ocular information in a condition that the examinee opens both of the left and right eyes, or to acquire ocular information of one eye at one time by occluding the other eye or turning off a fixation target.

The ophthalmological apparatus of the present disclosure can be formed into one that is capable of performing an arbitrary subjective or objective examination. The subjective examination is an examination in which the examinee is presented with an optotype or the like at a predetermined presentation position to obtain examination results based on the examinee's response to the presented optotype or the like. For example, the subjective examination includes the subjective refraction measurements such as a far-point examination, a mid-point examination, a near-point examination, a contrast test, a night test, a glare test, a pinhole test, a stereoscopic test and the like, and a visual field test and the like. The objective examination is an examination in which the subject eye E is irradiated with light and then, based on the detection result of the reflected light, ocular information regarding the subject eye E (eye characteristics thereof) is acquired. The objective examination includes the measurement for the acquisition of the eye characteristics of the subject eye E and capturing for the acquisition of an image (e.g., an anterior-ocular-segment image) of the subject eye E. For example, the objective examination includes objective refraction measurement (refraction measurement), cornea shape measurement (kerato-measurement), eye pressure measurement, fundus photography or fundus capturing, capturing with optical coherence tomography (referred to as OCT hereinafter), measurement with OCT, and the like.

In the first embodiment, as shown in FIG. 1, as viewed from the side facing the examinee, the leftward and rightward (left-right) directions indicated by the arrows X and the upward and downward (up-down or vertical) directions indicated by the arrows Y are the X-axis and Y-axis directions, respectively. The frontward and backward (front-back) directions, which are orthogonal to the left-right and up-down directions and indicated by the arrows Z, are the Z-axis directions. Furthermore, in the left-right directions (X-directions), with respect to the side facing the examinee, the left side is a side toward which the leftward direction faces, and the right side is one the rightward direction faces. Furthermore, in the front-back directions (Z directions), the examinee's side is the front side, and the opposite side is the back side.

The ophthalmological apparatus according to the first embodiment is a three-dimensional fundus imaging apparatus, which includes a fundus camera (fundus camera unit 100) that acquires fundus images of the subject eye E, and an OCT (OCT unit 200) that acquires fundus tomographic images of the subject eye E. Herein, the fundus camera refers to a camera that images a fundus condition of retina, optic nerve, capillaries, etc. at the back of the subject eye E (see FIG. 3, etc.) and captures the resulting fundus images. The OCT refers to an optical coherence tomography that images a section of the retina existing at the fundus of the subject eye E by using interference of light and captures the resulting fundus tomographic images. The ophthalmological apparatus 1 according to the first embodiment is one that observes, photographs, and records anterior-ocular-segment images, fundus images, fundus tomographic images, etc. of the subject eye and provides them in the form of electronic images.

As shown in FIG. 1, the ophthalmological apparatus 1 according to the first embodiment is equipped with an ophthalmological apparatus body H that has a base 10, a measuring head 20, a face (head) support 30, a control panel 40, an acquisition optical system 50, a controller 60, and a driver 70. Furthermore, the ophthalmological apparatus 1 is equipped with an optical table T, a chair, etc. not shown in the drawings, as peripheral devices (moving members) that work together with the ophthalmological apparatus body H. The acquisition optical system 50 is received in a housing 21 of the measuring head 20, and the control panel 40 is attached to an upper part of the housing 21.

In addition to the above configuration, the ophthalmological apparatus 1 according to the first embodiment may suitably be equipped with a printer that prints measurement results in response to measurement completion signals or instructions from the examiner, etc., an output section that outputs the measurement results to an external memory, server, etc., an audio output section that reports the status of operation, and the like.

The optical table T has a top plate T1, a pillar(s) not shown in the drawings that supports the top plate T1 in a manner to make its vertical movement possible, an optical table driver 74 to move the top plate T1 upwardly or downwardly, and a switch (not shown in the drawings) for operating the optical table driver 74, and the like.

The base 10 is formed on its top surface with the measuring head 20 that is made movable in the X-axis, Y-axis, and Z-axis directions. The base 10 is placed on the optical table T. The base 10 incorporates therein the controller 60, the driver 70, etc. Furthermore, the base 10 is equipped with an external input/output terminal 11 for a connection to an external memory or a personal computer, a power inlet (not shown in the drawings) for inserting a power cable, and a power switch (not shown in the drawings) for starting or stopping the ophthalmological apparatus 1.

The driver 70 moves a drive target member within a predetermined movable range. The driver 70 according to the first embodiment has an XYZ driver 71, a chin support driver 72, and an optical member driver 73, which are provided in the ophthalmological apparatus body H, and the above-mentioned optical table driver 74, which is provided to the optical table T, and the like. Each of the XYZ driver 71, the chin support driver 72, the optical member driver 73, and the optical table driver 74 is formed of a conventional motorized moving mechanism, which is composed of, for example, a motor actuator having a motor and a motor drive circuit. The above-mentioned movable range of the drive target member is the maximum movable range of each member to be moved by the motor. Each movable range is determined depending on the performance of the member to be moved, the motor, etc.

The XYZ driver 71 is built in the base 10 and moves the measuring head 20 in the X axis, Y axis and Z axis directions (hereinafter may be referred to as XYZ directions) relative to the base 10 under the control of the controller 60. Therefore, the XYZ driver 71 serves as an optical system driver that moves the acquisition optical system 50, which is received in the measuring head 20, in the XYZ directions in a predetermined movable range. The chin support driver 72 is provided at the face support 30 and moves the after-mentioned chin support 32 relative to the base 10 in vertical directions (Y axis directions) under the control of the controller 60. Therefore, the chin support driver 72 serves as a moving member driver that moves the chin support 32 as a moving member within a predetermined movable range.

The optical member driver 73 is built in the measuring head 20 and moves a movably disposed optical member(s) in the acquisition optical system 50 in a predetermined direction along the optical axis under the control of the controller 60 or rotates the same about a predetermined rotation axis. Therefore, the optical member driver 73 serves as one that moves the optical member(s) within a predetermined movable range. The optical member driver 73 according to the first embodiment is composed of a plurality of drive mechanisms that separately move a plurality of optical members to be moved.

The optical table driver 74 is provided to the optical table T and moves the top plate T1 of the optical table T in vertical directions (Y axis directions) under control of the controller 60. Therefore, the optical table driver 74 serves as a moving member driver that moves the top plate T1 as a moving member in a predetermined movable range. The optical table driver 74 can also be configured to make the top plate T1 movable in the left-right directions (X axis directions) or the front-back directions (Z axis directions), too.

The measuring head 20 is moved within a predetermined movable range by the XYZ driver 71 in the XYZ-axis directions. With this, the acquisition optical system 50, which is received in the measuring head 20, moves relative to the subject eye E back and forth, left and right, and up and down to change its position in the X-axis direction, the Y-axis direction and the Z-axis direction. That is, a positional relationship between the subject eye E and the acquisition optical system 50 is relatively changed by the XYZ driver 71.

The measuring head 20 is formed on its one surface of a side (front side) opposing to the examinee with a circular observation window 22 in which the after-mentioned objective lens 122 is fitted. The acquisition optical system 50 is received in the housing 21 such that it can be opposed to the subject eye through the observation window 22. The acquisition optical system 50 is installed in the housing 21 such that its optical path passes through the center of the observation window 22. In the vicinity of the observation window 22, a plurality (two in the first embodiment) of anterior-ocular-segment cameras 23 are provided. These anterior-ocular-segment cameras 23 are also included in the acquisition optical system 50.

A plurality of the anterior-ocular-segment cameras 23 are cameras that photograph an anterior ocular segment of the subject eye in different directions at substantially the same time and capture an anterior-ocular-segment camera image (anterior-ocular-segment image; see FIG. 5, etc.) of the subject eye. Each anterior-ocular-segment camera 23 is provided at a position away from the optical path of the acquisition optical system 50. In the first embodiment, two of the anterior-ocular-segment cameras 23 are disposed on the left and right sides to interpose the observation window 22 therebetween, but the anterior-ocular-segment cameras 23 can arbitrarily be designed in terms of their number, positions, etc. Considering the calculation process, however, it suffices that the anterior-ocular-segment cameras 23 have a configuration that makes it possible to photograph the anterior ocular segment from two different directions at substantially the same time. Herein, the phrase "at substantially the same time" means that the timings of capturing by the anterior-ocular-segment cameras 23 are allowed to slightly deviate from each other to the extent that eyeball movement can be ignored. With this, it is possible to capture an anterior-ocular-segment camera image by a plurality of the anterior-ocular-segment cameras 23 when the subject eye is at substantially the same position or in substantially the same direction.

Furthermore, the anterior-ocular-segment cameras 23 may be used to capture either moving images or still images. In the case of capturing moving images, it is possible to achieve the above-mentioned anterior-ocular-segment capturings at substantially the same time by a control to start the capturings at the same time or by a control of the capturing timing of the frame rate or each frame. In the case of capturing still images, it can be achieved by a control to start the capturings at the same time. An anterior-ocular-segment image Ga (see FIG. 5) captured by the anterior-ocular-segment cameras 23 is displayed on a display screen 41 of a control panel 40 by a control of the controller 60.

The face support 30 is fixed in front of the base 10. The face support 30 has a pillar 31 standing from the base and extending in the Y-axis direction, the chin support 32 provided on the pillar 31 to be movable in the Y-axis direction, and a forehead support 33 fixed to the top of the pillar 31. The face support 30 has a fixation-light holding section 34 for holding an external fixation light not shown in the drawings.

When acquiring ocular information of the subject eye E, the chin support 32 and the forehead support 33 fix the position of the subject eye E by supporting the face of the examinee facing the measuring head 20 (acquisition optical system 50). The chin support 32 is a place on which the examinee places his/her chin. The chin support 32 is moved in the Y-axis directions relative to the base 10 by the chin support driver 72. The forehead support 33 is a place against which the examinee places his/her forehead.

The external fixation light held by the fixation-light holding section 34 has a light source, at a tip of a flexible arm (not shown in the drawings), which emits fixation light to be used for external fixation. The light source of the external fixation light is arbitrarily adjustable in terms of its position and emission direction. By adjusting the position of the light source of the external fixation light, etc., the ophthalmological apparatus 1 can rotate the subject eye in any desired direction or can rotate the same to a greater extent than during internal fixation. If internal fixation cannot be performed, the ophthalmological apparatus 1 can use external fixation to guide the line of sight of the subject eye or fellow eye and adjust the orientation of the subject eye.

The ophthalmological apparatus 1 according to the first embodiment conducts examination, observation, image capturing, etc. of the subject eye in a condition that the examinee faces the measuring head 20 by placing his/her chin on the chin support 32 and his/her forehead against the forehead support 33 and that the external fixation light or internal fixation light is suitably turned on.

The control panel 40 has a display screen 41. The display screen 41 displays the anterior-ocular-segment image Ga (see FIG. 5, etc.) acquired by the anterior-ocular-segment cameras 23, and ocular information (for example, measurement results, anterior-ocular-segment observation images, etc.) acquired by the acquisition optical system 50. The display screen 41 according to the first embodiment is a touch-panel display screen with touch sensors stacked on the surface of a color liquid crystal panel. The examiner as an operator of the ophthalmological apparatus 1 can perform input operations to the controller 60 by touching button images, other images, etc. displayed on the display screen 41 with a finger. In other words, the display screen 41 also serves as an operation portion 42. The operation portion 42 may be configured by various buttons provided on the base 10 or the measuring head 20, input devices, such as a keyboard and mouse, connected to the measuring head 20, a card reader, a personal computer, etc.

The control panel 40 is rotatably attached to an upper rear part of the housing 21 of the measuring head 20 by a connecting support 43. The connecting support 43 has a support structure as a combination of (a) a rotary support that is capable of setting the display screen 41 relative to the measuring head 20 at any position in the circumferential direction and (b) a bending support that is capable of freely setting the inclination angle of the display screen 41 relative to the measuring head 20. Therefore, the examiner can dispose the control panel 40 at a position easy for the examiner to operate the display screen 41 wherever the examiner is at any position around the ophthalmological apparatus 1. As a result, the examiner can suitably perform operation of the operation portion 42 and observation of the display screen 41 not only at a position facing the examinee, but also at a position adjacent to the examinee side by side, etc.

FIG. 5 shows an exemplary screen design displayed on the display screen 41. As shown in FIG. 5, the display screen 41 has a first display region 41a, a second display region 41b, a third display region 41c, and an examinee's ID display region 41d. The first display region 41a displays therein, for example, the anterior-ocular-segment image Ga captured by the anterior-ocular-segment cameras 23. The second display region 41b displays therein, for example, a fundus image Gf acquired by an imaging optical system 102, a projection image (frontal image) generated by the OCT unit 200, etc. The third display region 41c displays therein, for example, a fundus tomographic image Gt generated by the OCT unit 200, etc. The first to third display regions 41a, 41b and 41c can separately and freely be set in terms of their size and arrangement in the display screen 41. The examinee's ID display region 41d displays therein the examinee's ID (patient ID) entered from the operation portion 42.

The display screen 41 displays, on the right side of the first display region 41a, a chin support vertical movement button 41e as an operation portion 42 for moving the chin support 32 in vertical directions. Furthermore, the display screen 41 displays therein necessary operation buttons 41f other than the chin support vertical movement button 41e, for suitably displaying more text information, operation methods, etc.

The ophthalmological apparatus 1 can also be equipped with a remote operation controller to enable the examiner to remotely operate the ophthalmological apparatus 1 from a position away from the examinee or the ophthalmological apparatus body H. The remote operation controller has input operation functions equivalent to those of the control panel 40 and a function to communicate with the ophthalmological apparatus body H. For the remote operation controller, it is possible to use a mobile terminal (information processing device) such as a tablet terminal or smartphone, a personal computer, a special controller, etc. The remote operation controller is communicatively connected to the controller 60 via a cable, a short-distance wireless connection such as Wi-Fi (registered trademark), or a communication network such as the Internet.

The acquisition optical system 50 is an optical system that acquires the anterior-ocular-segment image Ga, the fundus image Gf, the fundus tomographic image Gt, etc. as ocular information of the subject eye E. As shown in FIG. 3, the acquisition optical system 50 has a fundus camera unit 100 and an OCT unit 200. Configuration of the acquisition optical system 50 is not limited to that shown in FIG.3.

The configuration and the functions of the fundus camera unit 100 are explained as follows, with reference to FIG. 3. The fundus camera unit 100 acquires fundus images by photographing the fundus Ef of the subject eye E. The fundus image Gf to be acquired is a frontal image such as an observation image obtained by a moving image capturing using a near-infrared light or a still image obtained by using a flashlight. The fundus camera unit 100 can acquire a frontal image (anterior-ocular-segment image Ga) by photographing the anterior ocular segment Ea of the subject eye E.

The fundus camera unit 100 has an illumination optical system 101 that irradiates the subject eye E with an illumination light and an imaging optical system 102 that detects a return light of the illumination light from the subject eye E. A measurement light from the OCT unit 200 is guided to the subject eye E through an optical path in the fundus camera unit 100, and its return light is guided to the OCT unit 200 through the same optical path.

Light (observation illumination light) output from an observation light source 111 of the illumination optical system 101 is reflected by a reflection mirror 112 with a curved reflection surface, and becomes a near-infrared light after passing through a visible cut filter 114 via a first condenser lens 113. Further, the observation illumination light is once converged near an imaging light source 115, is reflected by a mirror 116, and passes through a first relay lens 117, a second relay lens 118, a first diaphragm 119 and a third relay lens 120. Then, the observation illumination light is reflected on a peripheral part (a surrounding region of an aperture part) of an aperture mirror 121, penetrates a first dichroic mirror 146, and is refracted by an objective lens 122, thereby illuminating the subject eye E (fundus Ef or anterior ocular segment Ea). A return light of the observation illumination light from the subject eye E is refracted by the objective lens 122, penetrates the first dichroic mirror 146, passes through the aperture part formed in a center region of the aperture mirror 121, and passes through a second dichroic mirror 155. The return light that has passed through the second dichroic mirror 155 travels through a focusing lens 131 and is reflected by a second mirror 132. Further, this return light passes through a half-mirror 133A, is reflected by a third dichroic mirror 133, and forms an image on a light receiving surface of a first image sensor 135 by a second condenser lens 134. The first image sensor 135 detects the return light at a predetermined frame rate. The imaging optical system 102 is adjusted to be focused on the fundus Ef or anterior ocular segment Ea. This focusing is conducted by moving the focusing lens 131 within a predetermined movable range in the directions of arrows shown in FIG. 3 by the optical member driver 73.

The light (imaging illumination light) output from the imaging light source 115 is applied to the fundus Ef via a route similar to that of the observation illumination light. The return light of the imaging illumination light from the subject eye E is guided to the third dichroic mirror 133 via the same route as that of the observation illumination light, passes through the dichroic mirror 133, is reflected by a third mirror 136, and forms an image on a light receiving surface of a second image sensor 138.

An LCD (Liquid Crystal Display) 139 displays a fixation target or a visual target for measuring visual acuity. Part of the light flux output from the LCD 139 is reflected by the half-mirror 133A, is reflected by the second mirror 132, travels through the focusing lens 131 and the second dichroic mirror 155, and passes through the aperture part of the aperture mirror 121. The light flux that has passed through the aperture part of the aperture mirror 121 penetrates the first dichroic mirror 146, and is refracted by the objective lens 122, thereby being projected onto the fundus Ef.

It is possible to change a fixation position of the subject eye E by changing a display position of the fixation target on the screen of the LCD 139. Examples of the fixation position include a fixation position for acquiring an image centered on the macula, a fixation position for acquiring an image centered on the optic papilla (optic disc), a fixation position for acquiring an image centered on the fundus center between the macula and the optic papilla, a fixation position for acquiring an image of a part (fundus periphery) far away from the macula, and the like. The ophthalmological apparatus 1 according to the first embodiment has GUI (Graphical User Interface), etc. for designating at least one of such fixation positions. The ophthalmological apparatus 1 according to the first embodiment has GUI, etc. for manually moving the fixation position (display position of the fixation target).

The configuration for presenting the movable fixation target to the subject eye E is not limited to display devices such as LCD. For example, the movable fixation target can be generated by selectively turning on a plurality of light sources in a light source array (light emitting diode array). Furthermore, the movable fixation target can be generated by at least one movable light source.

Furthermore, the fixation target can also be at least one external fixation light held on the fixation-light holding section 34. One of the at least one external fixation light can project the fixation light onto the fellow eye of the subject eye E. The projection position of the fixation light in the fellow eye is changeable. By changing the projection position of the fixation light against the fellow eye, the fixation position of the subject eye E can be changed. The fixation position by the external fixation light may be similar to that of the subject eye E using LCD 139. For example, the movable fixation target can be generated by selectively turning on one of the external fixation lights. Furthermore, the movable fixation target can be generated by the movable at least one external fixation light.

An alignment optical system 150 generates an alignment target used for an optical system alignment with respect to the subject eye E. An alignment light output from a first LED 151 travels through second diaphragms 152, 153 and a fourth relay lens 154, is reflected by a second dichroic mirror 155, and passes through the aperture part of the aperture mirror 121. The light that has passed through the aperture part of the aperture mirror 121 penetrates the first dichroic mirror 146 and is projected onto the cornea of the subject eye E by the objective lens 122. The cornea reflection light of the alignment light passes through the same route as that of the return light of the observation illumination light and is guided to the first image sensor 135. Based on the resulting optical image (alignment target image), a manual alignment or an automatic alignment is performed.

A focus optical system 160 generates a split target used for a focus adjustment against the subject eye E. The focus optical system 160 is moved within a predetermined movable range by the optical member driver 73, in directions of arrows shown in FIG. 3 along the optical path (illumination optical path) of the illumination optical system 101, with the movement of the focusing lens 131 along the optical path (imaging optical path) of the imaging optical system 102. A reflection rod 167 can be inserted into and removed from the illumination optical path by the optical member driver 73. When conducting focus adjustment, a reflection surface of the reflection rod 67 is arranged in a slanted position in the illumination optical path. Focus light output from a second LED 161 passes through a fifth relay lens 162, is split into two light fluxes by a split target plate 163, passes through a two-hole diaphragm 164, is reflected by a fourth mirror 165, and reflected after an image is once formed on the reflection surface of the reflection rod 167 by a fourth condenser lens 166. Further, the focus light travels through the third relay lens 120, is reflected by the aperture mirror 121, penetrate the first dichroic mirror 146, and is refracted by the objective lens 122, thereby being projected onto the fundus Ef. The fundus reflection light of the focus light passes through the same route as that of the cornea reflection light of the alignment light and is guided to the first image sensor 135. Based on the received light image (split target image), a manual focusing or automatic focusing is performed.

The first dichroic mirror 146 synthesizes the optical path for fundus photography and the optical path for OCT. The first dichroic mirror 146 reflects light of the wavelength band used in OCT and transmits light for fundus photography. In order from the OCT unit 200 toward the first dichroic mirror 146, the OCT light path (light path of the measurement light) is provided with a collimator lens unit 140, an optical-path-length changing portion 141, an optical scanner 142, an OCT focusing lens 143, a fifth mirror 144, and a sixth relay lens 145.

The optical-path-length changing portion 141 includes a first corner cube 141a. The optical-path-length changing portion 141 (first corner cube 141a) is movable in a predetermined movable range in the directions of arrows shown in FIG. 3 by the optical member driver 73, thereby changing the length of the OCT light path. This change in the optical path length is used for correcting the optical path in accordance with the axial length of the eye, adjusting the interference state, etc.

The optical scanner 142 is disposed at a position that is optically conjugate to pupil of the subject eye E. The optical scanner 142 deflects the measurement light LS (see FIG. 4) that passes through the OCT light path. The optical scanner 142 is, for example, a galvano scanner that is capable of a two-dimensional scanning. Since the optical scanner 142 is configured to scan a scanning site by the measurement light LS in directions perpendicular to each other, it is possible to perform scanning by the measurement light LS in X-axis direction and Y-axis direction respectively independently. Further, it is possible to perform scanning along any locus on the XY surface by simultaneously controlling the directions of two galvano mirrors contained in the optical scanner 142. The direction (scanning position) of the galvano mirror is changed in a predetermined movable range by the optical member driver 73.

The OCT focusing lens 143 is moved in directions of arrows shown in FIG. 3 within a predetermined movable range along the light path of the measurement light LS (see FIG. 4) by the optical member driver 73, for conducting the focus adjustment of the OCT optical system. The controller 60 can coordinatingly control the movement of the focusing lens 131, the movement of the focus optical system 160, and the movement of the OCT focusing lens 143 by controlling the optical member driver 73.

Configuration and function of the OCT unit 200 are explained as follows, with reference to FIG. 4. The OCT unit 200 is equipped with an optical system for performing swept source OCT, as exemplarily shown in FIG. 4. This optical system includes an interference optical system. This interference optical system is configured to have a function (splitter) of splitting the light emitted from a wavelength-tunable light source (wavelength swept light source) into the measurement light and the reference light, a function (interference light generator) of generating an interference light by superposing the return light of the measurement light returned from the subject eye E and the reference light that has traveled through the reference optical path, and a function (detector) of detecting this interference light. The result of the detection (detection signal) obtained by the interference optical system is a signal representing a spectrum of the interference light and is sent to the controller 60.

The light source unit 201 includes, for example, a near-infrared tunable laser configured to vary the wavelengths of emitted light at high speed. The light L0 output from the light source unit 201 is guided to a first polarization controller 203 through a first optical fiber 202, and the polarization state is regulated. The light L0 after the polarization state regulation is guided to a first fiber coupler 205 by a second optical fiber 204 and then split into the measurement light LS and the reference light LR.

The reference light LR is guided through a third optical fiber 210 to a first collimator 211, is converted into a parallel light beam, travels through an optical-path-length correction member 212 and a dispersion compensation member 213, and is guided to a second corner cube (reference mirror) 214. The optical-path-length correction member 212 acts to match the optical path length of the reference light LR and the optical path length of the measurement light LS with each other. The dispersion compensation member 213 acts to equalize the dispersion characteristics of the reference light LR and those of the measurement light LS with each other. The second corner cube 214 is moved in directions of arrows shown in FIG. 4 within a predetermined movable range along the light path of the reference light LR by the optical member driver 73. By the movement of the second corner cube 214, the light path length of the reference light LR is changed.

The reference light LR that has passed through the second corner cube 214 travels through the dispersion compensation member 213 and the optical-path-length correction member 212, is converted from a parallel light beam to a convergent light beam by the second collimator 216, and is incident on a fourth optical fiber 217. The reference light LR incident on the fourth optical fiber 217 is guided to a second polarization controller 218, and the polarization state of the reference light LR is regulated. Then, the reference light LR is guided to an attenuator 220 through a fifth optical fiber 219, and the light amount of the reference light LR is regulated. Subsequently, the reference light LR is guided to a second fiber coupler 222 through a sixth optical fiber 221.

Meanwhile, the measurement light LS generated by the first fiber coupler 205 is guided through a seventh optical fiber 227 and is converted to a parallel light beam by the collimator lens unit 140. Then, the measurement light LS passes through the optical-path-length changing portion 141, the optical scanner 142, the OCT focusing lens 143, the fifth mirror 144, and the sixth relay lens 145. The measurement light LS that has passed through the sixth relay lens 145 is reflected by the first dichroic mirror 146, is refracted by the objective lens 122, and is incident on the subject eye E. The measurement light LS is scattered and reflected at various depth positions of the subject eye E. The return light of the measurement light LS from the subject eye E travels along the same route as that of the outward way in the opposite direction, is guided to the first fiber coupler 205, and then reaches the second fiber coupler 222 via an eighth optical fiber 228.

The second fiber coupler 222 superposes the measurement light LS incident through the eighth optical fiber 228 with the reference light LR incident through the sixth optical fiber 221, to generate an interference light. The second fiber coupler 222 splits the interference light at a predetermined ratio (e.g., 1:1) to generate a pair of interference lights LC. The pair of interference lights LC is guided to a detector 225 through a ninth optical fiber 223 and a tenth optical fiber 224.

The detector 225 includes, for example, a balanced photodiode. The balanced photodiode includes a pair of photodetectors for respectively detecting the pair of the interference lights LC. The balanced photodiode outputs the difference between the detection results obtained by these photodetectors. The detector 225 sends this output (detection signal) to DAQ (Data Acquisition System) 230.

A clock KC is supplied from the light source unit 201 to the DAQ 230. The clock KC is generated in the light source 201 in synchronization with the output timings of the respective wavelengths varied within a predetermined wavelength range by the wavelength-tunable-type light source. For example, the light source unit 201 applies an optical delay to one of two split lights obtained by splitting the light L0 of each output wavelength, and then generates the clock KC based on the detection result of the superposed light. The DAQ 230 uses the clock KC to perform sampling of the detection signal input from the detector 225. The DAQ 230 sends the result of the sampling of the detection signal from the detector 225 to the controller 60.

As mentioned above, the ophthalmological apparatus according to the first embodiment is provided, as an optical member for adjusting the optical-path-length difference by changing the optical path length, with both of the optical-path-length changing portion 141 (first corner cube 141a) that changes the length of the optical path (measurement optical path or measurement arm) of the measurement light LS, and the second corner cube 214 that changes the length of the optical path (reference optical path or reference arm) of the reference light LR. However, it may be provided with either the optical-path-length changing portion 141 or the second corner cube 214. The ophthalmological apparatus 1 may be configured to change the difference between the measurement optical path length and the reference optical path length by using an optical member other than those.

Since operations of the fundus camera unit 100 and the OCT unit 200 are similar to those described in JP 2020-25719 A, their detailed descriptions are omitted.

The controller 60 extracts a program stored in a storage 61 connected thereto or in a built-in internal memory 62 on, for example, a RAM (Random Access Memory) and comprehensively controls the operation of the ophthalmological apparatus 1 in accordance with operations, etc. on the operation portion 42. In the first embodiment, the storage 61 is configured by a ROM (read-only memory), EEPROM (electrically erasable programmable ROM), etc., and the internal memory 62 is the RAM, etc.

The storage 61 stores various parameters for acquiring ocular information, the acquired results, etc., in addition to computer programs. Furthermore, the storage 61 stores history information of the target position of each part when the examinee acquired his or her ocular information by the ophthalmological apparatus 1. This target position refers to a target position of each part when each part of the acquisition optical system 50 (optical members contained in the acquisition optical system 50), the chin support 32, the optical table T, etc. has been moved to take a proper or optimum position for acquiring ocular information by the ophthalmological apparatus 1. The above-mentioned history information of the target position refers to the target position of each part when the examinee acquired ocular information in the past (for example, the last time), that is, the previous target position.

The previous target position contained in the history information is, for example, a three-dimensional positional information (hereinafter referred to as XYZ position) of the measurement head 20 obtained when the measurement head 20 was moved in XYZ directions at alignment, etc., a positional information (hereinafter referred to as focusing position) of the focusing lens 131, etc. obtained when the focusing lens 131, etc. was moved along the optical axis at focusing, a positional information (hereinafter referred to as reference mirror position) of optical members obtained when at least one of the first corner cube 141a and the second corner cube 214 was moved at the optical-path-length difference adjustment, a positional information (hereinafter referred to as chin support position) of the chin support 32 obtained when the chin support 32 was moved in Y-axis direction, a positional information (hereinafter referred to as optical table position) of the optical table T obtained when the optical table T was moved in Y-axis direction, and the like. The target position is not limited to these, and can also include a positional information of the optical scanner, those of members that are moved or rotated by other drivers 70, and the like. Once acquiring ocular information, the history information may be updated by the acquired target position of each part. The history information when acquiring a plurality of ocular information may be stored in the storage 61 in chronological order.

The controller 60 controls operations of the anterior-ocular-segment cameras 23 to acquire the anterior-ocular-segment image and controls the chin support driver 72 and the optical table driver 74 to move the chin support 32 and the optical table T within predetermined movable ranges. The controller 60 controls the control panel 40 to display images on the display screen 41. The controller 60 controls the XYZ driver 71 to move the measurement head 20 (acquisition optical system 50) to a position (XYZ position) within a predetermined movable range, thereby performing alignment and tracking.

The controller 60 controls the fundus camera unit 100 and the OCT unit 200. For example, the controller 60 controls LCD 139 to display the fixation target on the screen or to continuously or stepwise change the display position of the fixation target. The controller 60 controls the optical member driver 73 to move the focusing lens 131 within a predetermined movable range along the optical axis of the imaging optical system 102 and to move the focusing optical system 160 within a predetermined movable range along the optical axis of the illumination optical system 101. With this, the focus position of the imaging optical system 102 is changed.

The controller 60 controls the optical member driver 73 to move the OCT focusing lens 143 within a predetermined movable range along the optical axis of the measurement optical path. With this, the focus position of the measurement light LS is changed. The focus position of the measurement light LS corresponds to the depth position (z-position) of beam waist of the measurement light LS.

The controller 60 controls the observation light source 111, the imaging light source 115, the first LED 151, the second LED 161, the first image sensor 135, the second image sensor 138, etc. of the illumination light system 101 in the fundus camera unit 100 to operate them. The controller 60 controls the optical member driver 73 to move the optical scanner 142 and the reflection rod 167 within a predetermined movable range. The controller controls the light source unit 201, the attenuator 220, the first polarization controller 203, the second polarization controller 218, the detector 225, DAQ 230, etc. of the OCT unit 200 to operate them.

The controller 60 controls the fundus camera unit 100, etc. to perform the fundus imaging. The controller 60 controls the fundus camera unit 100 and the OCT unit 200, etc. to perform the OCT measurement. The controller 60 can perform multiple preliminary operations before conducting the OCT measurement. Such preliminary operations include alignment, focus rough adjustment, focus fine adjustment, polarization adjustment, optical-path-length difference adjustment, etc.

Focus rough adjustment is conducted by using the above-mentioned split target. Focus fine adjustment is conducted, based on interference sensitivity of the OCT measurement. For example, the controller 60 can conduct the OCT measurement of the subject eye E to acquire an interference signal and monitor the interference strength (interference sensitivity), thereby determining the position of the OCT focusing lens 143 at which the interference strength becomes maximum, and then can move the OCT focusing lens 143 to that position by controlling the optical member driver 73, thereby conducting focus fine adjustment. Polarization adjustment is conducted by adjusting the polarization condition of the reference light LR in order to optimize the interference efficiency between the measurement light LS and the reference light LR.

The optical-path-length difference adjustment is conducted by the controller 60 controlling the optical member driver 73 to move the optical-path-length changing portion 141 and/or the second corner cube 214 such that a target spot of the subject eye E is displayed at a predetermined z-position in a frame of the OCT image. With this, the optical-path-length difference between the measurement optical path and the reference optical path is adjusted. As the target spot to be used as a reference for the optical-path-length difference adjustment, a spot showing a characteristic luminance in the OCT image (or a spot showing a characteristic reflection strength in a reflection strength profile) is set in advance. For example, in the fundus OCT measurement, retinal pigment epithelium can be set as the reference. In the anterior-ocular-segment OCT measurement, the cornea surface can be set as the reference. An automatic process to search for such a suitable optical-path-length difference is called auto-Z.

The optical-path-length difference adjustment is not limited to auto-Z. For example, the controller 60 is capable of performing an automatic process to maintain a suitable image display position achieved by auto-Z. Such process is called Z-lock. In Z-lock, the controller 60 controls the optical member driver 73 to move the optical-path-length changing portion 141 and/or the second corner cube 214, for example, in a manner to maintain a condition in which the target spot as the reference for the optical-path-length difference adjustment is displayed at a predetermined z-position in the frame.

In the first embodiment, the controller 60 can designate or specify the reference position (z-position) of Z-lock. That is, the optical-path-length difference adjustment is conducted to maintain a condition in which the target spot of the subject eye E is displayed at the designated reference position (or Z-lock position specified based on this reference position). Setting of the reference position of Z-lock is not limited to the above.

The controller 60 acquires the examinee's ID that is input from the operation portion 42. The examinee's ID is entered, for example, from a touch panel of the display screen 41 as the operation portion 42 or a keyboard, or by reading a medical examination ticket, etc. with a card reader. The examinee's ID is not limited to one entered from the operation portion 42. For example, it may be one entered from another ophthalmological apparatus to be connected with the ophthalmological apparatus 1, an administrator's terminal, etc. through a cable, a communication network, etc.

The controller 60 acquires history information of the examinee from the storage 61, based on the acquired examinee's ID. The history information is information including the target position of each part of the ophthalmological apparatus 1 when the examinee acquired ocular characteristics in the past. In the case of successfully having acquired history information corresponding to the examinee's ID, it means that the examinee acquired in the past his/her ocular information by the ophthalmological apparatus 1. In this case, the controller 60 determines a search range for searching a proper target position at which each part of the ophthalmological apparatus operates, based on the acquired history information. Based on the determined search range, the controller 60 controls each part, while changing setting of each part of the ophthalmological apparatus 1. Then, when the position of each part has taken a proper position (i.e., the present target position), it controls the acquisition optical system 50 to acquire ocular information. The controller 60 stores the target position of each part, when acquired the present ocular information, in the storage 61 with a link to the examinee's ID to update history information of the examinee.

More specifically, for example, the controller 60 acquires the previous XYZ position (history information) of the measurement head 20. Based on this previous XYZ position, the controller 60 determines a search range for searching a proper XYZ position of the measurement head 20 in the present ocular information acquisition. In other words, the controller 60 sets, based on the acquired previous XYZ position, its surrounding area (for example, the XYZ position ± several millimeters in each of the X, Y and Z-axis directions) as the search range. The controller 60 controls the XYZ driver 71 to move the measurement head 20 within this search range, thereby conducting the alignment. The controller 60 stores in the storage 61 the XYZ position of the measurement head 20 acquired when the alignment has been completed, thereby updating history information of the XYZ position.

The controller 60 acquires the previous reference mirror position (history information) of the first corner cube 141a and/or the second corner cube 214. Based on the acquired reference mirror position, the controller 60 determines its surrounding area as the search range. Based on this search range, the controller 60 controls the optical member driver 73 to move the optical-path-length changing portion 141 and/or the second corner cube 214 and change the optical path length of the measurement light LS and/or the reference light LR, thereby adjusting the optical-path-length difference. The controller 60 stores the reference mirror position of the first corner cube 141a and/or the second corner cube 214 in the storage 61 when acquired a proper optical-path-length difference, thereby updating history information of the reference mirror position.

On the other hand, in case that history information corresponding to the examinee's ID cannot be acquired or is not stored, it is the first acquisition of ocular information. Therefore, for example, the controller 60 sets a predetermined movable range of each part as the search range, and, based on this search range, controls each part such that each part takes the target position. When each part has taken the target position, the controller 60 controls the acquisition optical system 50 to acquire ocular information. The controller 60 stores in the storage 61 the target position of each part with a link to the examinee's ID when the present ocular information has been acquired, thereby newly registering history information of the examinee.

The controller 60 generates an OCT image of the subject eye E, based on the sampling data obtained by sampling the detection signal from the detector 225 of the OCT unit 200 at DAQ 230. The OCT image to be generated by the controller 60 includes A-scan image, B-scan image (fundus tomographic image), C-scan image, etc. Based on the OCT image, the controller 60 generates a three-dimensional image, etc. of the fundus Ef. An arithmetic process for forming these images is similar to the arithmetic process to be performed in conventional swept-source-type OCT devices. The controller 60 conducts various image processing and analysis processing on the generated OCT image, three-dimensional image, and images (fundus image, anterior-ocular-segment image, etc.) obtained by the fundus camera unit 100, and controls the control panel 40 to display these images on the display screen 41.

One example of operation in the ophthalmological apparatus 1 according to the first embodiment 1 is explained as follows, based on a flowchart shown in FIG. 8, but it is not limited to this operation. The operation shown in FIG. 8 starts when the examinee has placed his/her chin on the chin support 32 and has applied his/her forehead to the forehead support 33 to fix the position of his/her face (subject eye) in a condition that the ophthalmological apparatus 1 has been switched on. Meanwhile, the examiner enters the examinee's ID into the ophthalmological apparatus 1 by operating the operation portion 42.

In Step S1, the controller 60 acquires the examinee' ID entered from the operation portion 42. At the next Step 2, the controller 60 acquires history information of the target position from the storage 61, based on the examinee's ID. In case that the examinee acquired ocular information in the past by the ophthalmological apparatus 1 (hereinafter referred to as having examination history), history information corresponding to the examinee's ID is acquired. In contrast, in case that the examinee did not acquire ocular information in the past (hereinafter referred to as having no examination history), history information corresponding to the examinee's ID is not acquired. The history information includes, for example, the previous (the last time) XYZ position, focus position, reference mirror position, chin support position, optical table position, etc.

After acquiring history information in Step S2, the controller 60 may control the chin support driver 72 and the optical table driver 74, based on the acquired previous chin support position and optical table position, to conduct a rough adjustment of the heights of the chin support 32 and the optical table T.

In Step S3, the controller 60 controls the anterior-ocular-segment cameras 23 and the imaging optical system 102 of the fundus camera unit 100 to acquire, for example, the anterior-ocular-segment image and the fundus image of the subject eye E. Then, the controller 60 controls the control panel 40 to respectively display the acquired anterior-ocular-segment image Ga and fundus image Gf on the first and second display regions 41a, 41b and display the examinee's ID on the examinee's ID display region 41d (see FIG. 5).

The examiner conducts a touching operation of the chin support vertical movement button 41e while observing the display screen 41, in order to make a height adjustment of the chin support 32. In Step S4, the controller 60 controls the chin support driver 72 in accordance with the touching operation against the chin support vertical movement button 41e, thereby conducting a height adjustment of the chin support 32 by moving the same in vertical directions. Upon this, the examiner may conduct a height adjustment of the optical table T (ophthalmological apparatus body H) by moving the top plate T1 together with the ophthalmological apparatus body H through operating a switch, etc. of the optical table T. The height adjustment of the chin support 32 and the optical table T and the height adjustment of the chair are not limited to those operated by the examiner, but the controller 60 can also be configured to determine respective search ranges based on the previous chin support position, optical table position, and chair position, and to control the chin support driver 72 and the optical table driver 74 based on those search ranges to move the chin support 32 or the optical table T in Y-axis directions, such that the anterior-ocular-segment image captured by the anterior-ocular-segment cameras 23, etc. takes a predetermined position.

Next, the examiner designates or specifies a scan area at a desired position in the fundus image Gf displayed in the second display region 41b by conducting a predetermined operation against the operation portion 42. Upon receiving the operation input to the operation portion 42, in Step S5, the controller 60 sets a scan area for the subject eye E. This scan area is a region on the subject eye E as a scan object of the measurement light LS, that is, a region on the subject eye E as an object of the OCT measurement.

In Step S6, the controller 60 determines a search range for searching the present XYZ position, based on the previous XYZ position of the measurement head 20. In case that the examinee has no examination history (i.e., the first examination), as shown in FIG. 6(1), the controller 60 sets a range that is the same as or slightly narrower than the movable range as a search range (Search Range 1). In contrast, in case that the examinee has examination history (reexamination), based on the previous XYZ position, the controller 60 sets a range of the previous XYZ position ± several millimeters in each of X-axis, Y-axis and Z-axis directions as a search range (Search Range 2), as shown in FIG. 6(1). That is, Search Range 2 is narrower than the movable range of the measurement head 20 and still narrower than Search Range 1.

In Step S7, the controller 60 performs alignment, based on the search range (Search Range 1 or Search Range 2) of the XYZ position determined in Step S6. For example, the controller 60 controls the alignment optical system 150 to project an alignment target onto the subject eye E. Upon this, the alignment optical system 150 also projects a fixation target onto the subject eye E by LCD 139. The controller 60 controls the XYZ driver 71 within the search range, based on a quantity (vector) of the movement of the measurement head 20 specified based on the received image acquired by, for example, the first image sensor 135, thereby moving the measurement head 20 (acquisition optical system 50) relative to the subject eye E by the quantity of the movement. The controller 60 repeatedly performs this process while changing the quantity of the movement, thereby moving the measurement head 20 to the target XYZ position. With this, alignment is completed. The controller 60 can also perform alignment based on the anterior-ocular-segment image Ga captured by the anterior-ocular-segment cameras 23. This enables a more precise alignment.

Herein, in the first examination, as shown in FIG. 6(1), while changing the quantity of the movement within Search Range 1, alignment is performed to reach the target XYZ position. In contrast, in the reexamination, based on the previous XYZ position, while changing the quantity of the movement within Search Range 2 that is narrower than the movable range and Search Range 1, alignment is performed to reach the target XYZ position. Therefore, the ophthalmological apparatus 1 according to the first embodiment can shorten the period of time necessary for alignment in the reexamination, as compared with the first examination.

After completing alignment, in the next Step S8, the controller 60 determines a search range for searching the present reference mirror position, based on the previous reference mirror position, in order to perform auto-Z (optical-path-length difference adjustment). In the reexamination, this search range is also set to be narrower than the movable range and still narrower than the search range at the first examination of the first corner cube 141a and/or the second corner cube 214.

In the next Step S9, the controller 60 performs auto-Z, based on the search range of the reference mirror position determined in Step S8. That is, the controller 60 starts the OCT measurement. In this OCT measurement, substantially the same profile of the subject eye E (e.g., fundus Ef) is repeatedly scanned at a predetermined frequency. Subsequently, similar to the method disclosed in JP 2016-041221 A, auto-Z is performed based on the acquired OCT image (or the reflection strength profile, etc.). Herein, the controller 60 controls the control panel 40 to live display the acquired OCT image on the display screen 41.

In this case too, the search range of the reference mirror position in the reexamination can be narrower than the movable range of the first corner cube 141a and/or the second corner cube 214 and still narrower than the search range in the first examination of the same. Thus, the search time in the reexamination can be shorter than that in the first examination. Therefore, the ophthalmological apparatus 1 can shorten the time necessary for auto-Z. The controller 60 may perform auto-Z after moving the focusing lens 131 to the previous focus position by controlling the optical member controller 73. With this, it is possible to more appropriately and more quickly perform auto-Z.

In the next Step S10, the controller 60 determines a search range for searching the present focus position (position of the focusing lens 131), based on the previous focus position. In the reexamination, this search range is also set to be narrower than the movable range and still narrower than the search range in the first examination of the focusing lens 131, etc.

As shown in FIG. 6(2), according to the claimed invention, the search range is divided into search divisions at predetermined intervals, and it is also possible to make a configuration that the search for the focus position is performed by intermittently or discontinuously setting or changing the quantity of the movement (movement position) at the predetermined intervals, and that the focusing lens 131 is moved based on this quantity of the movement. In this case, it is not only possible to make Search Range 2 in the reexamination narrower than Search Range 1 in the first examination, but also possible to make Search Division 2 in the reexamination narrower than Search Division 1 in the first examination.

In Step S11, the controller 60 starts focus adjustment by searching the focus position, based on the search range of the focus position determined in the above Step S10. For example, the controller 60 makes the fundus camera unit 100 start acquisition of a front image of the fundus Ef and controls the focus optical system 160 to project a split target onto the fundus Ef. The controller 60 controls the optical member driver 73, based on a quantity of the movement of the optical system specified based on the received image acquired by, for example, the first image sensor 135, thereby moving the focusing lens 131 by the quantity of the movement. Herein, the controller 60 controls the optical member driver 73 to move the OCT focusing lens 143 by the quantity of the movement. The controller 60 repeatedly performs this process while changing quantity of the movement within the search range, thereby moving the focusing lens 131 and the OCT focusing lens 143 to the target focus positions. With this, focusing is completed.

In this case too, as shown in FIG. 6(2), Search Range 2 of the focus position in the reexamination is narrower than the movable range of the focusing lens 131 and the OCT focusing lens 243 and still narrower than Search Range 1 of the focus position in the first examination. Therefore, the ophthalmological apparatus 1 can further shorten the time necessary for focusing. Furthermore, in case that the search division (Search Division 2) in the reexamination is made narrower than the search division (Search Division 1) in the first examination, the ophthalmological apparatus 1 can more precisely search the focus position with a shorter period of time, thereby conducting focusing more appropriately.

The controller 60 may perform a plurality of preliminary operations in alignment of Step S7 or focusing of Step S11. The preliminary operations include alignment, focusing rough adjustment, optical-path-length difference adjustment, polarization adjustment, focusing fine adjustment, etc.

In Step S12, the controller 60 controls the OCT unit 200, etc. to perform an OCT preliminary measurement. Subsequently, the controller 60 forms a projection image of the subject eye E.

In Step S13, the controller 60 designates or specifies the reference position for Z-lock (artifact specification). In other words, the controller 60 specifies an artifact caused by fold-over, based on the projection image formed in the above Step S12, and sets the reference position of Z-lock, corresponding to the displayed position of the specified artifact, relative to a predetermined B-scan. In case that the artifact is not specified in this Step S13, the Z-lock reference position is set to a predetermined value relative to a predetermined B-scan.

In Step S14, the controller 60 acquires the Z-lock position. That is, the controller 60 acquires the Z-lock position relative to other B-scans, based on the Z-lock reference position designated in Step S13.

In Step S15, the controller 60 acquires the quantity of change of the optical-path-length difference, for example, based on the Z-lock position acquired in the above Step S14, in order to perform B-scans in sequence. Then, the controller 60 controls the optical member driver 73, based on the acquired quantity of change of the optical-path-length difference, to move at least one of the first and second corner cubes 141a, 214.

In Step S16, the controller 60 controls the optical member driver 73 to control the optical scanner 142, thereby deflecting the measurement light LS and performing B-scan relative to the subject eye E.

In Step S17, the controller 60 generates a fundus tomographic image Gt, based on the scan result obtained in Step S16. In the next Step S18, the controller 60 controls the control panel 40 to display the fundus tomographic image Gt generated in Step S17, in the third display region 41c of the display screen 41.

In Step S19, the controller 60 determines if the next B-scan is performed or not. In other words, the controller 60 determines if the next B-scan is performed or not, based on the scan area set in Step S5 or on the operation contents or details against the operation portion 42.

If determined to perform the next B-scan (YES in Step S19), operation of the ophthalmological apparatus 1 goes to the above Step S15, and the procedures of Steps S15 to S18 are repeated. In contrast, if determined not to perform the next B-scan (NO in Step S19), operation of the ophthalmological apparatus 1 goes to Step S20.

In Step S20, the controller 60 generates a projection image (front image), based on the scan data of the scan area set in the above Step S5. In the next Step S21, the controller 60 controls the control panel 40 to display the projection image generated in the above Step S20 by overwriting the fundus image Gf of the second display region 41b of the display screen 41.

In Step S22, the controller 60 stores the target positions (the XYZ position acquired in the above Step S7, the focus position acquired in the above Step S9, the reference mirror position acquired in the above Step S11, the chin support position, the optical table position, etc.) in the storage 61 with a link to the examinee's ID and updates his/her history information. In the first examination, the controller 60 stores these target positions in the storage 61 with a link to the examinee's ID and newly registers his/her history information. With this, operation of the ophthalmological apparatus 1 is completed (END).

As explained above, the ophthalmological apparatus 1 according to the first embodiment
includes an acquisition optical system 50 that is configured to acquire ocular information of a subject eye E of an examinee; a driver 70, 71, 72, 73, 74 that is configured to move the acquisition optical system 50 relative to the subject eye E within a predetermined movable range; a controller 60 that is configured to control the driver 70, 71, 72, 73, 74 such that the acquisition optical system 50 is positioned at a target position; and a storage 61 that is configured to store a history information of the target position with a link to an ID of the examinee. The controller 60 is configured to acquire the ID of the examinee and then, based on the acquired ID of the examinee, acquire the history information from the storage 61. The controller 60 is configured to determine a search range for searching the target position, based on the history information. The controller 60 is configured to control the driver 70, 71, 72, 73, 74, based on the search range, such that the acquisition optical system 50 is positioned at the target position.

This ophthalmological apparatus 1 according to the claimed invention is configured to determine the search range of the XYZ position, based on history information of the previous XYZ position, in order to move the acquisition optical system 50 and position the same at a proper target position (XYZ position), and can shorten the search time. As a result, the ophthalmological apparatus 1 according to the first embodiment can shorten the period of time necessary for the position adjustment of each part, thereby efficiently acquiring ocular information.

As mentioned above, the ophthalmological apparatus 1 according to the first embodiment does not just move the target member to the previous target position itself, but moves the target member within a search range determined based on history information to determine the present target position. Therefore, even if the XYZ position, the focus position, the reference mirror position, the chin support position, the optical table position, etc. of the examinee have deviated from their appropriate or previous positions due to eyesight change, progress of disease, growth or aging of the examinee, etc., it is possible to search their appropriate target positions and shorten the search time. As a result, the ophthalmological apparatus 1 can more appropriately and more efficiently acquire ocular information.

In the ophthalmological apparatus 1 according to the claimed invention, the search range is within and narrower than the movable range of the acquisition optical system 50. Therefore, the ophthalmological apparatus 1 can shorten the period of time necessary for searching the target position of the acquisition optical system 50 and can more efficiently acquire ocular information.

In the ophthalmological apparatus 1 according to the first embodiment, the driver 70, 71, 72, 73, 74 includes an optical system driver 71 that is configured to perform at least one of a horizontal movement, a vertical movement, and a rotation around a rotational axis of the acquisition optical system 50, relative to the subject eye E. The controller 60 is configured to control the optical system driver 71, based on the search range, such that the acquisition optical system 50 is positioned at the target position. Therefore, the ophthalmological apparatus 1 can shorten the period of time necessary for the movement of the acquisition optical system 50 in XYZ directions. As a result, it is possible to shorten the period of time necessary for alignment. This search range determination of the acquisition optical system 50 is effective, when quantity of the movement of the acquisition optical system 50 cannot be specified, based on the received image acquired by the first image sensor 135 (for example, when it cannot be specified due to a failure to acquire the subject eye's image by the first image sensor 135).

In the ophthalmological apparatus 1 according to the first embodiment, the driver 70, 71, 72, 73, 74 includes an optical member driver 73 that is configured to move an optical member (for example, the focusing lens 131, the focus optical system 160, the OCT focusing lens 143, the first and second corner cubes 141a, 214, optical scanner 142, etc.) of the acquisition optical system 50 within a predetermined movable range. The controller 60 is configured to control the optical member driver 73, based on the search range, such that the optical member 131, 160, 143, 141a, 214, 142 is positioned at the target position (the focus position, the reference mirror position, and the scan position). Therefore, the ophthalmological apparatus 1 can shorten the period of time necessary for the movement of the optical member. As a result, it is possible to shorten the period of time necessary for focusing, optical-path-length difference adjustment, scan, etc.

In the ophthalmological apparatus 1 according to the first embodiment, the acquisition optical system 50 includes an interference optical system (OCT unit 200) including a light source (light source unit 201); a splitter (first fiber coupler 205) that is configured to split a light from the light source 201 into a measurement light LS and a reference light LR; an optical-path-length changing portion (the first and/or second corner cubes 141a, 214) that is configured to change or adjust an optical path length of at least one of a measurement optical path of the measurement light LS and a reference optical path of the reference light LR by a movement within a predetermined movable range; a measurement optical system (OCT unit 200) that is configured to irradiate the subject eye E with the measurement light LS that has passed through the measurement optical path; and a detector 225 that is configured to detect an interference light LC formed between a return light from the subject eye E and the reference light LR that has passed through the reference optical path. The optical member driver 73 is configured to move the optical-path-length changing portion 141a, 214 to the target position. Therefore, the ophthalmological apparatus 1 can shorten the period of time necessary for the movement of the optical member of the interference optical system. As a result, it is possible to shorten the period of time necessary the optical-path-length difference adjustment.

In the ophthalmological apparatus 1 according to the first embodiment, the ophthalmological apparatus 1 further includes a movable member (for example, the chin support 32, the optical table T, etc.) that is movable relative to the examinee or the acquisition optical system 50. The driver 70, 71, 72, 73, 74 includes a movable member driver (for example, the chin support driver 72 and the optical table driver 74) that is configured to move the movable member 32, T within a predetermined movable range. The controller 60 is configured to control the movable member driver 72, 74, based on the search range, such that the movable member 32, T is positioned at the target position. Therefore, the ophthalmological apparatus 1 can shorten the period of time necessary for the movement of the movable member. As a result, it is possible to shorten the period of time necessary for the height adjustment of the chin support 32, the optical table T, etc.

In the ophthalmological apparatus 1 according to the first embodiment, the search range is divided into search divisions at predetermined intervals, and the controller 60 is configured to search the target position by adjusting a range of each search division. Therefore, it is possible to shorten the period of time necessary for searching the target position of each member (particularly the target position of the focus position of the focusing lens 131). By adjusting the range of each search division, the search time can be shortened, and the target position is more precisely determined. By setting the range of each search division in the reexamination to be shorter than that in the first examination, it is possible to further shorten the search time.

As above, the ophthalmological apparatus according to the first embodiment of the present disclosure has been described. However, the specific configuration of the present disclosure is not limited to the first embodiment. Changes and additions in design should be allowed as long as they do not deviate from the gist of the inventions recited in the claims.

In the ophthalmological apparatus according to the first embodiment, the acquisition optical system 50 (measuring head 20) is movable in X-axis, Y-axis and Z-axis directions, but may further be rotatable around X-axis, Y-axis, Z-axis, or other axes. With this configuration, the ophthalmological apparatus can determine search range for conducting the movement or rotation of each of the left and right acquisition optical systems 50. As a result, the ophthalmological apparatus can shorten the period of time necessary for alignment of the left and right acquisition optical systems 50, focusing, the optical-path-length difference adjustment, etc. and shorten the period of time for acquiring ocular information, thereby more efficiently acquiring ocular information.

The controller 60 can also be configured to analyze the anterior-ocular-segment image Ga prepared by synthesizing images captured by the anterior-ocular-segment cameras 23 to acquire a three-dimensional positional information of the inner and outer corners of the subject eye E to detect inclination of the examinee's face. Even in case that the face is inclined, the controller 60 can more precisely conduct alignment etc. by correcting quantity of deviation caused by inclination, based on the detected inclination of the face. Upon this, as shown in FIG. 7, the controller 60 displays the synthesized anterior-ocular-segment image Ga in the first display region 41a and at the same time displays two horizontal line L by superimposing them on upper and lower edges of the anterior-ocular-segment image Ga. By observing this image, the examiner can recognize inclination of the subject eye E, that is, inclination of the examinee's face (head) and can recognize that the examinee's face is not fixed in a proper posture by the face support 30. Therefore, the examiner can persuade the examinee into fixing his/her face again to the face support 30 in a proper manner or move the examinee's head to a proper position. As a result, ocular information is acquired more appropriately and more efficiently.

The controller 6 may be configured to display an error message on the display screen 41 or outputs voice, etc. to prepare for a condition that the examinee's face is too inclined to properly conduct the correction. This configuration makes it possible to accurately inform the examiner and the examinee that the examinee's face is excessively inclined. As a result, it is possible to properly correct the face position to acquire ocular information more appropriately and more efficiently. In particular, in the case of remotely operating the ophthalmological apparatus 1, it is effective to display an error message or output voice.

As a modification of the first embodiment, the controller 60 can be configured to output history information of the target positions in the present first ophthalmological apparatus 1 to another second ophthalmological apparatus or a server. This second ophthalmological apparatus may determine the search range of the target position of each part, based on history information acquired directly from the first ophthalmological apparatus 1 or through the server. With this, for example, even if the examinee receives the first examination by the second ophthalmological apparatus, it is possible to send history information from the first ophthalmological apparatus 1 to the second ophthalmological apparatus. Thus, it is also possible even in the second ophthalmological apparatus to shorten the period of time necessary for searching the target position of each part and more efficiently acquire ocular information by using history information from the first ophthalmological apparatus 1. The search range of the target position may be determined depending on the type of the subject eye or examinee (e.g., height, age, gender, etc.), in addition to history information described in the present disclosure.

## Claims

1. An ophthalmological apparatus (1) comprising:
an acquisition optical system (50) that is configured to acquire ocular information of a subject eye (E) of an examinee, the acquisition optical system (50) being received in a housing (21) of a measurement head (20);
a driver (70, 71, 72, 73, 74) that is configured to move the acquisition optical system (50) relative to the subject eye (E) within a movable range;
a controller (60) that is configured to control the driver (70, 71, 72, 73, 74) such that the acquisition optical system (50) is positioned at a target position; and
a storage (61) that is configured to store a history information of the target position with a link to an ID of the examinee;
wherein the controller (60) is configured to acquire the ID of the examinee and then, based on the acquired ID of the examinee, acquire the history information from the storage (61),
wherein the controller (60) is configured to determine a search range for searching the target position, based on the history information, and
wherein the controller (60) is configured to control the driver (70, 71, 72, 73, 74), based on the search range, such that the acquisition optical system (50) is positioned at the target position,
**characterized in that**
in case that the examinee has no examination history, the controller (60) is configured to set a range that is the same as or slightly narrower than the movable range as the search range,
and **in that** in case that the examinee has examination history, based on a previous XYZ position of the measurement head (20) contained in the history information, the controller (60) is configured to set a range of the previous XYZ position ± several millimeters in each of X-axis, Y-axis and Z-axis directions as the search range,
wherein the search range is divided into search divisions at predetermined intervals,
wherein the controller (60) is configured to search the target position by changing a vector of movement of the driver (70, 71, 72, 73, 74) at the intervals,
wherein the search division in case that the examinee has examination history is made narrower than the search division in case that the examinee has no examination history.

2. The ophthalmological apparatus (1) according to claim 1, wherein the driver (70, 71, 72, 73, 74) comprises an optical system driver (71) that is configured to perform at least one of a horizontal movement, a vertical movement, and a rotation around a predetermined rotational axis of the acquisition optical system (50), relative to the subject eye (E).

3. The ophthalmological apparatus (1) according to claim 1, wherein the driver (70, 71, 72, 73, 74) comprises an optical member driver (73) that is configured to move an optical member (131, 160, 143, 141a, 214, 142) of the acquisition optical system (50) within a predetermined movable range.

4. The ophthalmological apparatus (1) according to claim 3, wherein the acquisition optical system (50) includes an interference optical system (200) comprising:
a light source (201);
a splitter (205) that is configured to split a light from the light source (201) into a measurement light (LS) and a reference light (LR);
an optical-path-length changing portion (141a, 214) that is configured to change an optical path length of at least one of a measurement optical path of the measurement light (LS) and a reference optical path of the reference light (LR) by a movement within a predetermined movable range;
a measurement optical system (200) that is configured to irradiate the subject eye (E) with the measurement light (LS) that has passed through the measurement optical path; and
a detector (225) that is configured to detect an interference light (LC) formed between a return light from the subject eye (E) and the reference light (LR) that has passed through the reference optical path,
wherein the optical member driver (73) is configured to move the optical-path-length changing portion (141a, 214) to the target position.

5. The ophthalmological apparatus (1) according to claim 1, wherein the ophthalmological apparatus (1) further comprises a movable member (32, T) that is movable relative to the examinee or the acquisition optical system (50),
wherein the driver (70, 71, 72, 73, 74) comprises a movable member driver (72, 74) that is configured to move the movable member (32, T) within a predetermined movable range.

## Patentansprüche

1. Ophthalmologische Vorrichtung (1), umfassend:
ein optisches Erfassungssystem (50), das konfiguriert ist, Augeninformationen eines Patientenauges (E) zu erfassen, wobei das optische Erfassungssystem (50) in einem Gehäuse (21) eines Messkopfes (20) aufgenommen ist;
einen Antrieb (70, 71, 72, 73, 74), der konfiguriert ist, das optische Erfassungssystem (50) relativ zu dem Patientenauge (E) innerhalb eines Bewegungsbereichs zu bewegen;
eine Steuerung (60), die konfiguriert ist, den Antrieb (70, 71, 72, 73, 74) so zu steuern, dass das optische Erfassungssystem (50) an einer Zielposition positioniert wird; und
einen Speicher (61), der konfiguriert ist, Verlaufsinformationen der Zielposition mit einer Zuordnung zu einer ID der zu untersuchenden Person zu speichern;
wobei die Steuerung (60) konfiguriert ist, die ID der zu untersuchenden Person zu erfassen und anschließend basierend auf der erfassten ID der zu untersuchenden Person die Verlaufsinformationen aus dem Speicher (61) abzurufen,
wobei die Steuerung (60) konfiguriert ist, basierend auf den Verlaufsinformationen einen Suchbereich für die Suche nach der Zielposition zu bestimmen, und
wobei die Steuerung (60) konfiguriert ist, den Antrieb (70, 71, 72, 73, 74) basierend auf dem Suchbereich so zu steuern, dass das optische Erfassungssystem (50) an der Zielposition positioniert wird,
**dadurch gekennzeichnet, dass**
für den Fall, dass für die zu untersuchende Person keine Untersuchungshistorie vorliegt, die Steuerung (60) konfiguriert ist, einen Bereich, der gleich oder geringfügig enger ist als der Bewegungsbereich, als den Suchbereich festzulegen, und dass
für den Fall, dass für die zu untersuchende Person eine Untersuchungshistorie vorliegt, die Steuerung (60) konfiguriert ist, basierend auf einer früheren XYZ-Position des Messkopfes (20), die in den Verlaufsinformationen enthalten ist, einen Bereich der früheren XYZ-Position von ± mehreren Millimetern in jeder der Richtungen der X-Achse, der Y-Achse und der Z-Achse als den Suchbereich festzulegen,
wobei der Suchbereich in vorbestimmten Intervallen in Suchabschnitte unterteilt wird,
wobei die Steuerung (60) konfiguriert ist, die Zielposition durch Änderung eines Bewegungsvektors des Antriebs (70, 71, 72, 73, 74) in den Intervallen zu ermitteln,
wobei der Suchabschnitt in dem Fall, dass für die zu untersuchende Person eine Untersuchungshistorie vorliegt, enger gefasst wird als der Suchabschnitt in dem Fall, dass für die zu untersuchende Person keine Untersuchungshistorie vorliegt.

2. Ophthalmologische Vorrichtung (1) nach Anspruch 1, wobei der Antrieb (70, 71, 72, 73, 74) einen Antrieb (71) für ein optisches System umfasst, der konfiguriert ist, mindestens eine der folgenden Bewegungen auszuführen: eine horizontale Bewegung, eine vertikale Bewegung und eine Rotation um eine vorbestimmte Rotationsachse des optischen Erfassungssystems (50) relativ zu dem Patientenauge (E).

3. Ophthalmologische Vorrichtung (1) nach Anspruch 1, wobei der Antrieb (70, 71, 72, 73, 74) einen Antrieb (73) für ein optisches Element umfasst, der konfiguriert ist, ein optisches Element (131, 160, 143, 141a, 214, 142) des optischen Erfassungssystems (50) innerhalb eines vorbestimmten Bewegungsbereichs zu bewegen.

4. Ophthalmologische Vorrichtung (1) nach Anspruch 3, wobei das optische Erfassungssystem (50) ein optisches Interferenzsystem (200) aufweist, umfassend:
eine Lichtquelle (201);
einen Strahlteiler (205), der konfiguriert ist, ein Licht von der Lichtquelle (201) in ein Messlicht (LS) und ein Referenzlicht (LR) aufzuteilen;
einen Abschnitt (141a, 214) zur Veränderung der optischen Weglänge, der konfiguriert ist, eine optische Weglänge eines optischen Messwegs des Messlichts (LS) und/oder eines optischen Referenzwegs des Referenzlichts (LR) durch eine Bewegung innerhalb eines vorbestimmten Bewegungsbereichs zu verändern;
ein optisches Messsystem (200), das konfiguriert ist, das Patientenauge (E) mit dem Messlicht (LS) zu bestrahlen, das den optischen Messweg durchlaufen hat; und
einen Detektor (225), der konfiguriert ist, ein Interferenzlicht (LC) zu detektieren, das zwischen einem vom Patientenauge (E) zurückgeworfenen Licht und dem Referenzlicht (LR), das den optischen Referenzweg durchlaufen hat, gebildet wird,
wobei der Antrieb (73) für das optische Element konfiguriert ist, den Abschnitt (141a, 214) zur Veränderung der optischen Weglänge zu der Zielposition zu bewegen.

5. Ophthalmologische Vorrichtung (1) nach Anspruch 1, wobei die ophthalmologische Vorrichtung (1) ferner ein bewegliches Element (32, T) umfasst, das relativ zu der zu untersuchenden Person oder dem optischen Erfassungssystem (50) bewegbar ist,
wobei der Antrieb (70, 71, 72, 73, 74) einen Antrieb (72, 74) für das bewegliche Element umfasst, der konfiguriert ist, das bewegliche Element (32, T) innerhalb eines vorbestimmten Bewegungsbereichs zu bewegen.

## Revendications

1. Appareil ophtalmologique (1) comprenant:
un système optique d'acquisition (50) configuré pour acquérir des informations oculaires relatives à l'œil du sujet (E) d'une personne examinée, le système optique d'acquisition (50) étant logé dans un boîtier (21) d'une tête de mesure (20) ;
un dispositif d'entraînement (70, 71, 72, 73, 74) configuré pour déplacer le système optique d'acquisition (50) par rapport à l'œil du sujet (E) dans une plage de déplacement;
un contrôleur (60) configuré pour commander le dispositif d'entraînement (70, 71, 72, 73, 74) de telle sorte que le système optique d'acquisition (50) soit positionné à une position cible; et
un stockage (61) configuré pour stocker des informations d'historique de la position cible, associées à un identifiant de la personne examinée;
le contrôleur (60) étant configuré pour acquérir l'identifiant de la personne examinée, puis, sur la base de l'identifiant acquis de la personne examinée, acquérir les informations d'historique à partir du stockage (61),
le contrôleur (60) étant configuré pour déterminer une plage de recherche permettant de localiser la position cible, sur la base des informations d'historique, et
le contrôleur (60) étant configuré pour commander le dispositif d'entraînement (70, 71, 72, 73, 74), sur la base de la plage de recherche, de telle sorte que le système optique d'acquisition (50) soit positionné à la position cible,
**caractérisé en ce que**
dans le cas où la personne examinée n'a pas d'historique d'examen, le contrôleur (60) est configuré pour définir comme plage de recherche une plage identique à la plage de déplacement ou légèrement plus étroite que la plage de déplacement, et **en ce que**
dans le cas où la personne examinée a un historique d'examen, sur la base d'une position XYZ antérieure de la tête de mesure (20) contenue dans les informations d'historique, le contrôleur (60) est configuré pour définir comme plage de recherche une plage correspondant à la position XYZ antérieure ± plusieurs millimètres dans chacune des directions des axes X, Y et Z,
la plage de recherche étant divisée en sections de recherche à des intervalles prédéterminés,
le contrôleur (60) étant configuré pour rechercher la position cible en modifiant, à ces intervalles, un vecteur de mouvement du dispositif d'entraînement (70, 71, 72, 73, 74),
la section de recherche, lorsque la personne examinée a un historique d'examen, étant plus restreinte que la section de recherche lorsque la personne examinée n'a pas d'historique d'examen.

2. Appareil ophtalmologique (1) selon la revendication 1, le dispositif d'entraînement (70, 71, 72, 73, 74) comprenant un dispositif d'entraînement du système optique (71) qui est configuré pour effectuer au moins l'un parmi un mouvement horizontal, un mouvement vertical et une rotation autour d'un axe de rotation prédéterminé du système optique d'acquisition (50), par rapport à l'œil du sujet (E).

3. Appareil ophtalmologique (1) selon la revendication 1, le dispositif d'entraînement (70, 71, 72, 73, 74) comprenant un dispositif d'entraînement d'élément optique (73) qui est configuré pour déplacer un élément optique (131, 160, 143, 141a, 214, 142) du système optique d'acquisition (50) dans une plage de déplacement prédéterminée.

4. Appareil ophtalmologique (1) selon la revendication 3, le système optique d'acquisition (50) comprenant un système optique à interférence (200) comprenant:
une source lumineuse (201);
un séparateur (205) configuré pour diviser la lumière provenant de la source lumineuse (201) en une lumière de mesure (LS) et une lumière de référence (LR) ;
une partie de modification de la longueur du trajet optique (141a, 214) qui est configurée pour modifier la longueur du trajet optique d'au moins l'un parmi un trajet optique de mesure de la lumière de mesure (LS) et un trajet optique de référence de la lumière de référence (LR) par un déplacement dans une plage de déplacement prédéterminée ;
un système optique de mesure (200) configuré pour irradier l'œil du sujet (E) avec la lumière de mesure (LS) ayant traversé le trajet optique de mesure ; et
un détecteur (225) configuré pour détecter une lumière d'interférence (LC) formée entre une lumière de retour provenant de l'œil du sujet (E) et la lumière de référence (LR) ayant traversé le trajet optique de référence,
le dispositif d'entraînement de l'élément optique (73) étant configuré pour déplacer la partie de modification de la longueur du trajet optique (141a, 214) vers la position cible.

5. Appareil ophtalmologique (1) selon la revendication 1, l'appareil ophtalmologique (1) comprenant en outre un élément mobile (32, T) pouvant se déplacer par rapport à la personne examinée ou au système optique d'acquisition (50),
le dispositif d'entraînement (70, 71, 72, 73, 74) comprenant un dispositif d'entraînement de l'élément mobile (72, 74) qui est configuré pour déplacer l'élément mobile (32, T) dans une plage de déplacement prédéterminée.
